# EUROPÄISCHE PATENTANMELDUNG

(11) **EP 1 958 586 A1**
(43) Veröffentlichungstag der Anmeldung: **20.08.2008**
(21) Anmeldenummer: 07102641.3
(22) Anmeldetag: 19.02.2007
(51) Int. Cl.: A61B 19/00, A61F 2/46, G01P 15/00, H01H 35/14, A61F 2/48

(54) **Chirurgische Vorrichtung mit Stoßfeststeller**

(71) Anmelder: BrainLAB AG, 85622 Feldkirchen (DE)
(72) Erfinder: Millahn, Manuel, 80799 München (DE); Neubauer, Timo, 85586 Poing (DE)
(74) Vertreter: Schwabe - Sandmair - Marx

(57) **Zusammenfassung**

Die vorliegende Erfindung betrifft eine chirurgische Vorrichtung (10) mit einem Stoßfeststeller (12), wobei die chirurgische Vorrichtung ein Instrument (10) oder ein Implantat oder eine Markereinrichtung für chirurgische Navigationssysteme ist sowie eine Abfragevorrichtung zum Abfragen einer chirurgischen Vorrichtung (10), wobei die Abfragevorrichtung (20) ausgebildet ist, um Stoßereignisinformation von der chirurgischen Vorrichtung (10) abzufragen.

## Beschreibung

Die vorliegende Erfindung betrifft chirurgische Vorrichtungen mit Stoßfeststeller, um Stoßereignisse festzustellen, die die chirurgische Vorrichtung erlitten hat. Weiter betrifft sie eine Abfragevorrichtung, um Information über ein Stoßereignis von der chirurgischen Vorrichtung abzufragen sowie ein Navigationssystem mit der Abfragevorrichtung.

Chirurgische Vorrichtungen, wie zum Beispiel Instrumente, Implantate oder Markereinrichtungen für chirurgische Navigationssysteme sollten für einen Arzt verlässliche geometrische Eigenschaften haben, d.h. insbesondere formstabil sein. Durch einen Stoß, falls beispielsweise die chirurgische Vorrichtung auf den Boden fällt, kann sich jedoch die Form der chirurgischen Vorrichtung verändern. Falls eine chirurgische Vorrichtung einen Stoß erlitten hat, kann dies also auch ein Hinweis darauf sein, dass die chirurgische Vorrichtung insteril geworden ist und/oder eine geänderte Form hat, wodurch sie nicht mehr für einen chirurgischen Eingriff ohne Risiko verwendet werden kann.

Aufgabe der Erfindung ist es eine chirurgische Vorrichtung bereitzustellen, bei der auf einfache Weise feststellbar ist, ob das Risiko einer Formveränderung und/oder einer Insterilität erhöht ist. Weiter soll eine Abfragevorrichtung bereitgestellt werden, die es erlaubt das erhöhte Risiko festzustellen sowie ein Navigationssystem, das die Abfragevorrichtung einsetzt.

Vorstehende Aufgabe wird durch die Gegenstände der unabhängigen Ansprüche gelöst. Vorteilhafte Weiterbildungen gehen aus den Unteransprüchen hervor.

Bei der chirurgischen Vorrichtung gemäß der Erfindung handelt es sich insbesondere um Vorrichtungen, die bei einem chirurgischen Eingriff Anwendung finden und bei denen eine (ungewünschte) Formveränderung das Risiko des Eingriffs erhöhen würde. Beispiele sind ein Instrument, wie z.B. ein Messer, eine Biopsienadel, ein Injektionsgerät mit Einstechspitze, ein Bohrgerät usw. Die chirurgische Vorrichtung kann auch ein Implantat sein, wie beispielsweise ein künstliches Hüftgelenk. Sie kann auch eine Markereinrichtung sein, wie sie für chirurgische Navigationssysteme verwendet wird. Bei derartigen Markersystemen ist eine definierte relative Lage zwischen Markerelementen (z.B. Markerkugeln) der Markereinrichtung wichtig. Diese Lage kann sich durch einen Stoß verändern. Beispiele für Markereinrichtungen sind Referenzsterne, bei denen Markerkugeln durch starre Stege eine vorbestimmte relative Lage zueinander einnehmen.

Insbesondere betrifft die vorliegende Erfindung die Verwendung des Stoßfeststellers zur Überprüfung der Formbeständigkeit der chirurgischen Vorrichtung und/oder zur Feststellung des Risikos einer Formveränderung.

Die chirurgische Vorrichtung umfasst insbesondere einen Transponder, z. B. einen RFID-Transponder, einen Karten-Transponder oder Funk-Transponder. Insbesondere umfasst die chirurgische Vorrichtung einen Transponder, insbesonder RFID-Transponder oder Mikrochip-Transponder, auf dem die geometrischen Eigenschaften der chirurgischen Vorrichtung gespeichert sind, die insbesondere die Form der chirurgischen Vorrichtung beschreiben. Im folgenden ist beispielhaft der RFID-Transponder genannt. Vergleiche hierzu die europäische Patentanmeldung Nr. 06 019 346.3. Vorzugsweise sind die geometrischen Eigenschaften (geometrische Daten) zusammen mit dem Datum der Messung der geometrischen Eigenschaften abgespeichert. Werden die geometrischen Eigenschaften ausgelesen und der Stoßfeststeller abgefragt, so werden die geometrischen Eigenschaften dann als ungültig angesehen, falls der Stoßfeststeller einen Stoß entsprechender Stärke anzeigt, der insbesondere nach der Kalibrierung oder Vermessung der chirurgischen Vorrichtung aufgetreten ist.

Der Stoßfeststeller dient der Feststellung eines Stoßereignisses. Er kann passiv, d.h. ohne eigene Stromversorgung ausgebildet sein und seinen Zustand aufgrund eines Stoßes, dessen Stärke insbesondere eine bestimmte Schwelle überschreitet, ändern. Dieser Zustand stellt ein Beispiel für eine Stoßereignisinformation dar und kann dann abgefragt werden. Er kann auch aktiv ausgebildet sein, d.h. eine eigene Stromversorgung umfassen. Der aktive Stoßfeststeller kann insbesondere einen Beschleunigungssensor (auch Chrashsensor, Schocksensor oder Stoßsensor genannt) umfassen, der insbesondere zeitaufgelöst Beschleunigungsdaten liefert, die beispielsweise von einem Mikrochip oder Mikroprozessor erfasst werden können und als Daten (elektrisch) abgespeichert werden können. Auch können diese Sensoren einen Mittelwert oder Maximalwert der Beschleunigung erfassen und entsprechende Signale und/oder Daten abgeben. Die Beschleunigungsdaten stellen Beispiele für Stoßereignisinfonnation dar.

Vorzugsweise ist der Stoßsensor, den der Stoßfeststeller umfasst, so ausgebildet, dass er durch die Einwirkung eines Stoßes eine elektrische Eigenschaft ändert, die dann abgefragt werden kann. Bei einer besonderen Ausführungsform, ändert sich die elektrische und/oder mechanische Eigenschaft des Stoßsensors permanent, wenn die Stärke des Stoßes eine Schwelle überschreitet. Beispielsweise kann der Stoßsensor so gestaltet sein, dass ein Draht reißt, wenn die Stärke des Stoßes eine Schwelle überschreitet. Das Reißen des Drahtes führt zu einer starken Änderung der elektrischen Eigenschaften des Drahtes (starke Erhöhung des Widerstandes und/oder der Hochfrequenzresonanzeigenschaften). Die elektrische Eigenschaft stellt ein Beispiel für Stoßereignisinformation dar.

Der geänderte Widerstand kann dann durch eine elektrische Schaltung beispielsweise abgefragt werden. Alternativ kann die geänderte Hochfrequenzresonanzeigenschaft, die die Stoßereignissinformation darstellt, ähnlich wie bei oder entsprechend einem Transponder mittels Hochfrequenzwellen detektiert werden. Der Stoßfeststeller umfasst also vorzugsweise einen Transponder oder ist so mit einem Transponder gekoppelt, dass die geänderten elektrischen und/oder mechanischen Eigenschaften des Stoßfeststellers an den Transponder übermittelt werden oder die von Transpondern abfragbare Information ändern.

Die chirurgische Vorrichtung ist vorzugsweise so ausgebildet, dass der Stoßfeststeller entnehmbar und/oder austauschbar und/oder in seinem ursprünglichen Zustand (vor dem Stoß) zurücksetzbar ist. Dies ist insbesondere dann von Vorteil, wenn der Stoßfeststeller durch einen (ausreichend starken) Stoß seinen (elektrischen) Zustand permanent ändert. Dabei ist als Stoß jede Art von Ereignis anzusehen, die eine mechanische Belastung ist, die die chirurgische Vorrichtung beschädigen oder sogar zerstören kann. Die chirurgische Vorrichtung kann dann beispielsweise zu einer Wartungsfirma eingesandt werden. Dort kann der Stoßfeststeller z.B. in der Art einer Sicherung wieder "scharf" gemacht werden oder durch einen neuen Stoßfeststeller (ähnlich wie beim Sicherungsaustausch) ersetzt werden. Außerdem können die geometrischen Eigenschaften der chirurgischen Vorrichtung bei der Wartungsfirma neu vermessen werden, die chirurgische Vorrichtung kann also neu kalibriert werden. Die neu gemessenen Daten können dann beispielsweise auf einem Transponder, insbesondere RFID-Transponder gespeichert werden, der sich innerhalb der chirurgischen Vorrichtung befindet. Hierzu wird auch auf die Europäische Patentanmeldung Nr. 06 019 346.3 (US Patentanmeldung Nr. 60/826,973) hingewiesen. Dort ist eine Messvorrichtung zum Messen geometrischer Eigenschaften einer medizintechnischen Behandlungsvorrichtung beschrieben, wobei ein RFID-Schreibgerät die geometrischen Eigenschaften (geometrische Daten) der medizintechnischen Behandlungsvorrichtung in einem RFID-Transponder schreibt, der sich in der medizintechnischen Behandlungsvorrichtung befindet. Das so neu kalibrierte Gerät mit einem erneuerten oder neu scharf gemachten Stoßfeststeller kann somit wieder genutzt werden. Die chirurgische Vorrichtung der vorliegenden Erfindung kann die Merkmale der medizintechnischen Behandlungsvorrichtung der europäischen Patentanmeldung Nr. 06 019 346.3 oder der US-Patentanmeldung Nr. 60/826,973 aufweisen.

Wie oben ausgeführt, kann der Stoßfeststeller direkt, insbesondere elektrisch durch ein Abfragegerät abgefragt werden. Dazu sind beispielsweise an der chirurgischen Vorrichtung Elektroden vorgesehen, an die ein Abfragegerät angeschlossen werden kann, das beispielsweise den Widerstand des Stoßfeststellers oder seine Kapazität misst. Vorzugsweise erfolgt die Abfrage jedoch kontaktlos, beispielsweise mit einem elektrischen Wechselfeld, insbesondere Hochfrequenzfeld, das beispielsweise eine geänderte Resonanzfrequenz des Stoßfeststellers detektiert.

Gemäß einer weiteren Ausführungsform umfasst die chirurgische Vorrichtung Transponder, insbesondere einen RFID-Transponder, der beispielsweise geometrische Daten über die chirurgische Vorrichtung speichern kann (aber nicht muss). Der Transponder, insbesondere RFID-Transponder kann insbesondere dazu verwendet werden, die von einem Stoßfeststeller ermittelte Information über ein Stoßereignis zu speichern und/oder an ein Transponder-Lesegerät-Lesegerät, insbesondere RFID-Lesegerät zu übermitteln. Hierzu wird der Transponder, insbesondere RFID-Transponder vorzugsweise elektrisch mit dem Stoßfeststeller gekoppelt. Beispielsweise kann ein Stoßfeststeller Daten zu dem Transponder, insbesondere RFID-Transponder senden, die ein Stoßereignis widerspiegeln, oder der Transponder, insbesondere RFID-Transponder kann diese Daten von dem Stoßfeststeller abfragen. Diese Daten können dann von dem Transponder, insbesondere RFID-Transponder in einem Speicher gespeichert werden und bei Bedarf durch ein Transponder-Lesegerät, insbesondere ein RFID-Lesegerät ausgelesen werden.

Auch wenn der Stoßfeststeller passiv ausgebildet ist, so kann beispielsweise durch Änderung eines Widerstandswertes des Stoßfeststellers der Wert einer Speicherzelle, insbesondere der binäre Wert bei einem ausreichend starken Stoß geändert werden. Der geänderte Speicherinhalt der Speicherzelle wird dann wiederum von einem Transponder-Lesegerät, insbesondere RFID-Lesegerät ausgelesen, wodurch festgestellt werden kann, ob die chirurgische Vorrichtung einem Stoß ausgesetzt war oder nicht.

Die vorliegende Erfindung ist weiter auf eine Abfragevorrichtung gerichtet, die zum Abfragen der erfindungsgemäßen chirurgischen Vorrichtung ausgebildet ist, um insbesondere Stoßereignisinformation und/oder geometrische Daten, die in einem Transponder, insbesondere RFID-Transponder gespeichert sind und die geometrischen Eigenschaften der chirurgischen Vorrichtung repräsentieren, von der chirurgischen Vorrichtung abzufragen. Die Abfragevorrichtung umfasst weiter vorzugsweise eine Vergleichseinheit, die eine Stoßtoleranzinformation mit der abgefragten Stoßereignisinformation vergleicht. Die Stoßereignisinformation stellt beispielsweise Beschleunigungsdaten dar, die vom Stoßfeststeller aufgezeichnet wurden oder sie spiegelt den Zustand des Stoßfeststellers wider, falls dieser zum Beispiel binär ausgebildet ist und nur vermittelt, ob ein Stoßereignis stattgefunden hat oder nicht. Die abgefragte Stoßereignisznformation wird vorzugsweise von der Vergleichseinheit mit Stoßtoleranzinformation verglichen. Dies gilt insbesondere dann, wenn die Stoßereignisinformation Information über die Stärke des Stoßes und/oder der Beschleunigung enthält, die die chirurgische Vorrichtung erlitten hat. Derartige Stoßereignisinformation kann mit einer Stoßtoleranzinformation verglichen werden, um festzustellen, ob die Stärke des Stoßes oberhalb einer Schwelle war. Falls die Stärke des Stoßes oberhalb der Schwelle war, kann dann insbesondere von einem Risiko der Beschädigung oder Formveränderung der chirurgischen Vorrichtung ausgegangen werden. Insbesondere kann davon ausgegangen werden, dass die geometrischen Daten, die bei einer Ausführungsform zusätzlich aus dem Transponder, insbesondere RFID-Transponder der chirurgischen Vorrichtung ausgegeben werden, nicht mehr zuverlässig sind.

Vorzugsweise ist die Abfragevorrichtung so ausgebildet, dass sie zusätzlich zu der Stoßereignisinformation Identifikationsdaten aus der chirurgischen Vorrichtung ausliest. Die Identifikationsdaten können beispielsweise in einem Transponder, insbesondere RFID-Transponder gespeichert sein, der in der chirurgischen Vorrichtung vorgesehen ist. Basierend auf den ausgelesenen Identifikationsdaten kann dann beispielsweise eine Datenbank abgefragt werden, in der die Identifikationsdaten jeweils verschiedene Stoßtoleranzinformation zugeordnet ist. Die Stoßtoleranzinformation repräsentiert die Stärke eines Stoßwertes, bei dem beispielsweise noch nicht mit dem Risiko einer Formveränderung zu rechnen ist oder ab dem mit einem derartigen Risiko zu rechnen ist. Die Stoßtoleranzinformation wird vorzugsweise mittels der Identifikationsdaten den einzelnen Typen von chirurgischen Vorrichtung zugeordnet. Verschiedene Typen von chirurgischen Vorrichtungen können unterschiedliche Stoßtoleranzen haben. Auf diese Art und Weise lässt sich dann individuell, abhängig vom Typ der clairurgischen Vorrichtung bestimmen, ob das Risiko einer Verformung besteht oder nicht. Vorzugsweise umfasst die Abfragevorrichtung die vorgenannte Datenbank. Vorzugsweise umfasst die Abfragevorrichtung eine Warneinheit, die einen Benutzer warnt, falls das Ergebnis des Vergleichs, der von der Vergleichseinheit durchgeführt wird, ein Risiko der Verformung anzeigt, also eine Toleranzüberschreitung anzeigt. Insbesondere, falls der Transponder, insbesondere RFID-Transponder zusätzlich die geometrischen Daten der chirurgischen Vorrichtung speichert, kann, falls ein derartiges Risiko besteht, ein Auslesen oder Weitergeben der geometrischen Daten blockiert werden oder die geometrischen Daten können nur mit einem Warnhinweis kombiniert weitergegeben werden.

Vorzugsweise umfasst die vorliegende Erfindung weiter ein Navigationssystem, insbesondere zum Durchführen von IGS (image guided surgery). Das Navigationssystem umfasst vorzugsweise die erfindungsgemäße Abfragevorrichtung. Stellt diese eine Toleranzüberschreitung oder das Risiko einer Verformung fest, so gibt vorzugsweise das Navigationssystem ein Warnsignal aus und/oder blockiert eine Navigation der chirurgischen Vorrichtung, für die ein Risiko der Verformung festgestellt wurde. Auf diese Art und Weise kann verhindert werden, dass verformte chirurgische Vorrichtungen bei einem medizinischen Eingriff verwendet werden. Insbesondere kann verhindert werden, dass nicht mehr gültige geometrische Daten der chirurgischen Vorrichtungen durch das Navigationssystem zugeordnet werden, die auf Grund eines Stoßes ungültig geworden sind, aber dennoch in einem Transponder, insbesondere RFID-Transponder der chirurgischen Vorrichtung gespeichert sind und von der Abfragevorrichtung des Navigationssystems ausgelesen werden. Somit wird vermieden, dass fehlerbehaftete oder risikobehaftete geometrische Daten bei der Navigation der chirurgischen Vorrichtung verwendet werden. Insbesondere kann veranlasst werden, dass die geometrischen Eigenschaften der chirurgischen Vorrichtung neu vermessen und im Transponder, insbesondere RFID-Transponder neu gespeichert werden, bevor die chirurgische Vorrichtung wieder verwendet wird.

Im Folgenden werden Ausführungsformen der Erfindung beschrieben. Dabei können Merkmale unterschiedlicher Ausführungsformen miteinander kombiniert werden.

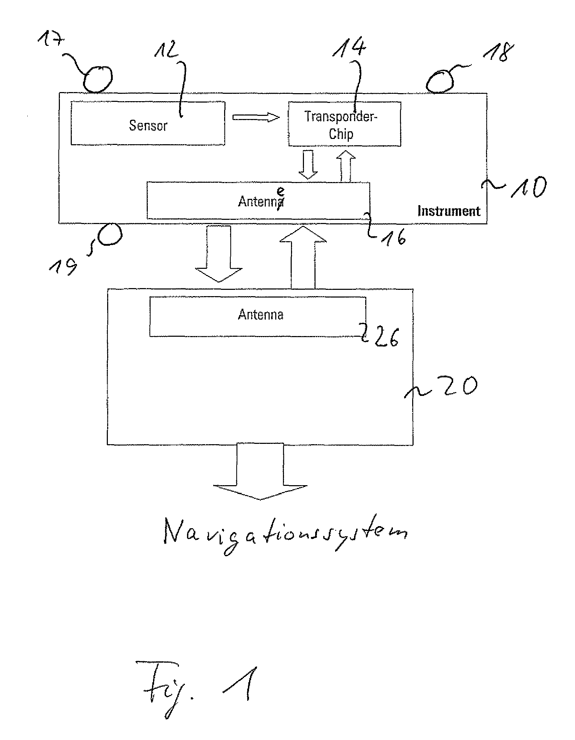

Figur 1 zeigt schematisch eine erfindungsgemäße chirurgische Vorrichtung und Abfragevorrichtung.

Figur 1 zeigt schematisch den Aufbau einer erfindungsgemäßen chirurgischen Vorrichtung 10 und einer Abfragevorrichtung 20. (Aktive oder passive) Markereinrichtungen (auch als Referenzeinheiten bezeichnet), z.B. Markerkugeln (passiv oder aktiv) 17, 18 und 19, die von einer Detektionseinrichtung (z.B. Kamera) eines Navigationssystems detektierbar sind, sind an der chirurgischen Vorrichtung angebracht. In dem gezeigten Beispiel ist die chirurgische Vorrichtung ein Instrument. Ein Stoßsensor 12 übermittelt Daten über ein Stoßereignis zum Beispiel auf elektrischem Weg zu einem Transponderchip 14 (zum Beispiel RFID-Tag). Der Transponderchip 14 empfängt Signale von einer Antenne 16 und gibt Signale zu der Antenne 16 aus. Die Antenne 16 steht in Wechselwirkung mit einem hochfrequenten elektromagnetischen Wechselfeld, das von einer Antenne 26 des Transponder-Lesegeräts, insbesondere des RFID-Lesegeräts 20 ausgesendet wird. Durch die Wechselwirkung können Signale zu dem Instrument 10, genauer zu dem Transponderchip 14 gesendet werden und Signale von dem Transponderchip 14 von dem Transponder-Lesegerät, insbesondere dem RFID-Lesegerät 20 empfangen werden. Das Transponder-Lesegerät, insbesondere das RFID-Lesegerät 20 kann die empfangenen Daten beispielsweise zu einem Navigationssystem weiterleiten oder kann Bestandteil eines derartigen Navigationssystems sein. Das Transponder-Lesegerät, insbesondere das RFID-Lesegerät 20 stellt ein Beispiel für die erfindungsgemäße Abfragevorrichtung dar. Der Sensor 12 kann beispielsweise ein Stoßsensor oder ein Beschleunigungsmesser sein. Beispiele für Stoßsensoren und ihre Anwendungen sind beispielsweise in folgenden Druckschriften beschrieben:
EP 1 258 896
JP 2004-093274
JP 2002-3 50459
JP 2001-289873
US 5,970,794
GB 2,424,916
US 5,731,957
US 2001-002451
DE 103 34 700
WO 2004049280
WO 03085617
WO 2005117691
WO 9924093
WO 0250554

Beispielsweise JP 2004-093274 und das entsprechende englische Abstract (Patent Abstracts of Japan) offenbart einen Beschleunigungssensor, der ohne Energieversorgung einen Stoßvorgang detektiert und aufgrund des Stoßvorganges seinen Zustand ändert. Genauer wird die Kapazität der Vorrichtung geändert, oder, falls ein piezoelektrisches Element verwendet wird, der Widerstand des piezoelektrischen Elements. Gemäß der dort beschriebenen Erfindung wird ein träges Massenteil von einem elastischen Schwenkmechanismus gehalten, der zwei Positionen einnehmen kann. Wird die Vorrichtung einer Beschleunigung ausgesetzt, so kann das träge Massenteil von der einen Position in die andere wechseln, wodurch sich insbesondere die elektrische Kapazität ändert, oder falls piezoelektrische Elemente entlang des elastischen Scharniers verwendet werden, der Widerstand der piezoelektrischen Elemente ändert. Der dort beschriebene Beschleunigungssensor kann wieder verwendbar gestaltet werden, falls das träge Massenteil wieder in die Ausgangsposition zurückgeführt wird. Der Transponderchip 14 kann beispielsweise auch die geometrischen Daten des Instruments 14 und/oder Identifikationsinformation, die das Instrument 14 identifiziert, speichern. Diese Daten bzw. Information kann ebenfalls von dem Transponder-Lesegerät, insbesondere dem RFID-Lesegerät 20 ausgegeben werden.

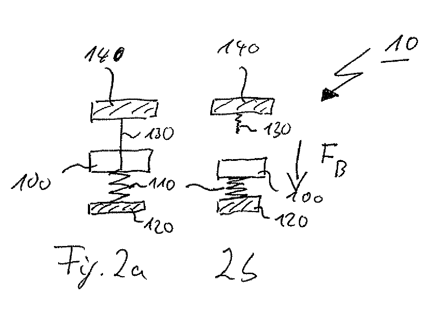

Figur 2a und 2b zeigt das Prinzip eines nicht wieder verwendbaren Stoßsensors. Ein träges Massenteil 100 ist über eine Feder 110 mit einer Elektrode 120 verbunden. In Figur 2a steht die Elektrode 120 über die Feder 110 und das träge Massenteil 100 mit einem Draht 130 in Verbindung, der gespannt ist. Der Draht 130 ist wiederum elektrisch verbunden mit der Elektrode 140, so dass ein Strom zwischen der Elektrode 140 und 120 fließen kann. Tritt eine Beschleunigungskraft F_{B} auf, wie in Figur 2b gezeigt wird, so wird das träge Massenteil 100 in Richtung auf die Elektrode 120 bewegt, so dass sich das elastische Element 110 komprimiert. Durch diese Bewegung reißt der Draht 130, der dadurch beispielsweise seine vorgegebene, ungespannte Ausdehnung einnimmt, die gegenüber der Ausdehnung in Figur 2a verkürzt ist. Somit ist die elektrische Verbindung zwischen der Elektrode 140 und 120 durch ein Stoßereignis, das die Beschleunigungskraft F_{B} auf das träge Massenteil 100 ausübt, unterbrochen. Der Stoßsensor gemäß Figur 2 ändert somit durch ein Stoßereignis seinen Widerstand. Dies kann wiederum von einem Transponderchip 14 oder auch allgemein von einem elektromagnetischen Wechselfeld abgefragt werden. Im letzteren Fall können beispielsweise an den Elektroden 120 und 140 Antennen angebracht werden, wobei sich die Resonanzeigenschaft der Antenne durch Reißen des Drahtes 130 ändert. Die geänderte Resonanzeigenschaft kann von einer Abfragevorrichtung, die Hochfrequenzwellen aussendet, detektiert werden kann. Die Detektion der geänderten Resonanzeigenschaft weist dann auf ein Stoßereignis hin, das das Instrument erlitten hat.

## Patentansprüche

1. Chirurgische Vorrichtung (10) mit Stoßfeststeller (12).

2. Chirurgische Vorrichtung nach Anspruch 1, wobei die chirurgische Vorrichtung ein Instrument (10) oder ein Implantat oder eine Markereinrichtung für chirurgische Navigationssysteme ist.

3. Chirurgische Vorrichtung nach einem der vorhergehenden Ansprüche, bei welcher der Stoßfeststeller (12) Stoßereignisinformation abrufbar speichert.

4. Chirurgische Vorrichtung nach einem der vorhergehenden Ansprüche, bei welchem der Stoßfeststeller passiv ausgebildet ist.

5. Chirurgische Vorrichtung nach einem der vorhergehenden Ansprüche, bei welcher der Stoßfeststeller ausgebildet ist, aufgrund eines Stoßes seine elektrische Eigenschaft permanent zu ändern, um so Stoßereignisinformation zu speichern.

6. Chirurgische Vorrichtung nach einem der Ansprüche 1 bis 3, bei welchem der Stoßfeststeller einen Beschleunigungssensor umfasst.

7. Chirurgische Vorrichtung nach einem der vorhergehenden Ansprüche, mit einem Transponder (14), der mit dem Stoßfeststeller so gekoppelt ist oder der so Bestandteil des Stoßfeststellers ist, dass sich die von dem Transponder (14) abfragbaren Daten durch einen Stoß ändern.

8. Chirurgische Vorrichtung nach einem der vorhergehenden Ansprüche, mit einem Speicher, dessen Inhalt sich mit der Änderung der elektrischen Eigenschaft des Stoßfeststellers ändert.

9. Chirurgische Vorrichtung nach einem der vorhergehenden Ansprüche, mit einem Speicher zum Speichern von Beschleunigungswerten.

10. Chirurgische Vorrichtung nach einem der vorhergehenden Ansprüche, mit einem Transponder (14), der ausgebildet ist, Stoßereignisinformation von einem Stoßfeststeller zu speichern und/oder an ein Lesegerät weiterzuleiten.

11. Chirurgische Vorrichtung nach einem der vorhergehenden Ansprüche mit einem RFID-Transponder (14), der geometrische Daten und/oder Identifikationsdaten der chirurgischen Vorrichtung gespeichert hat.

12. Abfragevorrichtung zum Abfragen einer chirurgischen Vorrichtung (10), wobei die Abfragevorrichtung (20) ausgebildet ist Stoßereignisinformation von der chirurgischen Vorrichtung (10) abzufragen.

13. Abfragevorrichtung nach Anspruch 12, mit einer Vergleichseinheit, die Stoßtoleranzinformation mit der abgefragten Stoßereignisinformation vergleicht.

14. Abfragevorrichtung nach Anspruch 12 oder 13, die ausgebildet ist, Identifikationsdaten von der chirurgischen Vorrichtung auszulesen und die weiter eine Stoßtoleranzinformations-Bereitstelluargseinheit umfasst, die Stoßtoleranzinformation entsprechend den Identifikationsdaten bereitstellt, wobei die Vergleichseinheit die bereitgestellte Stoßtoleranzinformation mit der abgefragten Stoßereignisinformation vergleicht.

15. Abfragevorrichtung nach einem der Ansprüche 12 bis 14 mit einer Warneinheit, die einen Benutzer warnt, falls das Vergleichsergebnis eine Toleranzüberschreitung anzeigt.

16. Abfragevorrichtung nach einem der Ansprüche 12 bis 15, wobei die Abfragevorrichtung weiter ausgebildet ist, geometrische Daten von einem Transponder der chirurgischen Vorrichtung auszulesen.

17. Navigationssystem mit einer Abfragevorrichtung nach einem der Ansprüche 11 bis 16, bei welchem bei Toleranzüberschreitung ein Warnhinweis gegeben wird und/oder eine Navigation der abgefragten chirurgischen Vorrichtung nicht unterstützt wird.

18. Navigationssystem nach Anspruch 17, bei welchem ausgelesene geometrische Daten der chirurgischen Vorrichtung nicht oder nur unter Warnhinweis verwendet werden, wenn die Stoßereignisinformation ein Stoßereignis anzeigt.

19. Verwendung eines Stoßfeststellers in einer chirurgischen Vorrichtung zur Überprüfung des Risikos einer Formveränderung der chirurgischen Vorrichtung.

20. System zur Überprüfung des Risikos einer Formveränderung einer chirurgischen Vorrichtung mit einer chirurgischen Vorrichtung nach einem der Ansprüche 1 bis 13 und einer Abfragevorrichtung nach einem der Ansprüche 14 bis 16 oder einem Navigationssystem nach Anspruch 17 oder 18.
